# EUROPÄISCHE PATENTSCHRIFT

(11) **EP 0 708 333 B1**
(45) Veröffentlichungstag und Bekanntmachung des Hinweises auf die Patenterteilung: **07.02.2001**
(21) Anmeldenummer: 95115747.8
(22) Anmeldetag: 06.10.1995
(51) Int. Cl.: G01N 33/36, G01N 3/08

(54) **Vorrichtung und Verfahren zum automatischen Bestücken eines Prüfapparates mit Stapelfaserproben**
Device and method for automatically providing a testing apparatus with samples of staple fibres
Dispositif et procédé pour transférer automatiquement des échantillons de fibres discontinues dans un appareil d'essai

(30) Priorität: 17.10.1994 DE 4437026
(43) Veröffentlichungstag der Anmeldung: 24.04.1996
(73) Patentinhaber: Trevira GmbH & Co KG, 60528 Frankfurt am Main (DE)
(72) Erfinder: Bader, Jochen, D-86830 Schwabmünchen (DE); Schneider, August, Dr., D-86845 Grossaitingen (DE); Stolarski, Erwin, D-86459 Wollishausen (DE)
(74) Vertreter: Luderschmidt, Schüler & Partner GbR

(56) Entgegenhaltungen:
- DE-A- 1 573 849
- DE-A- 1 648 802
- DE-A- 3 137 713
- DE-C- 4 343 157
- US-A- 3 063 294
- US-A- 5 178 007

## Beschreibung

Vorrichtung und Verfahren zum automatischen Bestücken eines Apparates zur Charakterisierung mechanischer und/oder geometrischer Eigenschaften von Stapelfaserproben

Die vorliegende Erfindung betrifft ein Magazin für Faserproben, welches das automatische Bestücken eines Apparates zum Charakterisierung von mechanischen und/oder geometrischen Eigenschaften von Stapelfaserproben, insbesondere von Kräuseleigenschaften, gestattet und ein Verfahren zum automatischen Bestücken eines derartigen Apparates unter Einsatz dieses Magazins.

Verfahren zur Charakterisierung von mechanischen und/oder geometrischen Eigenschaften von Fasern, wie zur Bestimmung der Kräuseleigenschaften von Fasern sind an sich bekannt.

Eine der Möglichkeiten, die Kräuseleigenschaften von Fasern zu charakterisieren, beruht auf der Bestimmung der sogenannten "Einkräuselung". Dazu wird die Faser zweimal mit Kräften vorgegebener Größe belastet, wobei die erste Kraft so gering gewählt wird, daß sie noch keine Einkräuselung bewirkt, und die zweite Kraft so groß gewählt wird, daß die Einkräuselung vollständig entfernt wird, ohne aber die Faser in Längsrichtung zu dehnen. Aus der Längendifferenz zwischen dem eingekräuselten Zustand und dem gestreckten Zustand der Faser wird eine als "Einkräuselung" bezeichnetete Kenngröße abgeleitet. Meßverfahren dieses Typs sind beispielsweise in der DE-A-2,925,810 beschrieben.

Zur Durchführung solcher Messungen hat sich die sogenannte "Kräuselwaage" eingebürgert. Dabei handelt es sich um eine Vorrichtung, in welche eine zu charakterisierende Stapelfaser an beiden Seiten eingeklemmt wird, wobei eine der Klemmen an einem Ende des Wägebalkens angebracht ist. Das andere Ende des Waagebalkens ist mit einer Vorrichtung für die Aufnahme eines Massestückes versehen. Dieses Massestück erzeugt die Kraft, die notwendig ist, um die zu prüfende Faser definiert zu entkräuseln.

Die zweite Klemme ist mittels Motorantrieb in der Faserachse beweglich ausgestaltet und sitzt auf der Welle einer Mikrometerschraube. Die Faser wird durch Verfahren der beweglichen Klemme so lange gestreckt, bis die von der Faser aufgenommene Zugkraft jener durch das Massestück am anderen Ende des Waagebalkens erzeugten Kraft entspricht.

Hiermit ist das Gleichgewicht der Waage hergestellt, was photoelektrisch erfaßt und mittels entsprechender Elektronik zum Stop der Klemmenbewegung benutzt wird.

Auf der Mikrometerschraube kann die dem Weg der Klemme entsprechende Längenänderung ΔL der Faser abgelesen werden.

Es sind bereits optische Messungen zur Bestimmung der Kräuseleigenschaften bekanntgeworden. So wird in der SU-A-1,183,898 eine optische Bestimmung des Kraft-Dehnungsverhaltens von gekräuselten Einzelfasern beschrieben. Aus der US-A-4,057,350 ist ein Verfahren zur Ermittlung der Einkräuselung von Faserkabeln bekannt, worin die Streuung eines Laserstrahls als Meßgröße für den Grad der Einkräuselung verwendet wird. Weitere optische Meßmethoden zur Bestimmung der Einkräuselung von laufenden Faserkabeln sind aus der WO-A-92-2,001, der US-A-4,270,252 und der RD-209,007 bekannt. Die DE-PS-1,473,750 beschreibt die Überwachung der Gleichförmigkeit der Kräuselung von Faserkabeln mittels einer mechanischen Methode.

Ferner ist es bekannt, zur Charakterisierung der Kräuselung von Fasern die Anzahl der Kräuselbögen pro Längeneinheit der Faser, bei vorgegebener Faserspannung, zu bestimmen. Zu diesem Zweck wird üblicherweise die Kräuselwaage herangezogen und es wird die Anzahl der Kräuselbögen der eingespannten Fasern visuell ausgewertet. Das bekannte Verfahren eignet sich nicht zur Automatisierung; außerdem ist es personal- und damit kostenintensiv.

Aus der DE-A-1,648,802 ist eine Einrichtung zum selbsttätigen Zuführen von Proben bei einer Zerreißmaschine für Textilfasern und dergleichen bekannt. Der Probenträger bei dieser Einrichtung ist tellerartig ausgestaltet.

Aus der DE-A-3,137,713 ist eine Meßvorrichtung mit selbsttätiger Beschickung und automatischem Arbeitszyklus zur Durchführung von Zugversuchen an Fasern bekannt. Diese Meßvorrichtung ist mit einem Magazin ausgestattet, welches in Spannstücke eingeklebte Faserproben aufweist. Diese Spannstücke werden zur Messung zusammen mit den Faserproben in die Meßvorrichtung übernommen.

Eine Weiterentwicklung des voranstehend beschriebenen automatisierten Meßverfahrens wird in der DE-C-3,829,197 offenbart, welche eine vereinfachte Probenvorbereitung sowie den Einsatz handelsüblicher, nicht auf das Messen von Einzelfasern abgestimmter Prüfgeräte gestattet. Nach dieser Schrift werden Einzelfasern zu einem Endlosfaden verbunden, der anschließend einem Prüfverfahren unterworfen wird.

Ein weiteres automatisiertes Verfahren zur Charakterisierung von mechanischen und/oder geometrischen Eigenschaften von Stapelfasern sowie eine zur Durchführung geeignete Vorrichtung sind in der nicht veröffentlichten Deutschen Patentanmeldung P 43 43 157.7 beschrieben.

Die vorliegende Erfindung betrifft eine Vorrichtung und ein Verfahren, mit denen der Automatisierungsgrad der Charakterisierung von mechanischen und/oder geometrischen Eigenschaften von Faserproben nochmals vergrößert werden kann.

Die vorliegende Erfindung betrifft eine Vorrichtung zum automatischen Bestücken eines Apparates (1) zur Charakterisierung mechanischer und/oder geometrischer Eigenschaften von Stapelfaserproben umfassend folgende Elemente:
A) mehrere Klemmleisten (2) die jeweils mehrere Klemmen (3) zur Aufnahme von Faserproben (4) aufweisen,
B) mindestens eine Vorratseinrichtung (5) für die Klemmleisten (2), und
C) eine Transportvorrichtung (6), mit der die Klemmleisten (2) oder einzelne Faserproben (4) von der Vorratseinrichtung (5) automatisch in den Apparat (1) verbracht werden.

Das Fixieren der Faserproben (4) auf den Klemmleisten (2) kann auf beliebige Art und Weise erfolgen, beispielsweise mittels mechanischer Methoden, wie mit Federn oder mit Gegengewichten, oder mit pneumatisch oder hydraulisch betriebenen Klemmen, oder mit magnetisch, wie permanent- oder elektromagnetisch betriebenen Klemmen.

Die Klemmleisten können in der Vorratseinrichtung (5) fest positioniert sein oder vorzugsweise bewegbar sein.

Besonders bevorzugt sind die Klemmleisten (2) in der Vorratseinrichtung (5) horizontal bewegbar. Die horizontale Bewegung der Klemmleisten (2) in der Vorratseinrichtung (5) kann beispielsweise erfolgen durch Kettentrieb oder durch pneumatisch oder hydraulisch betriebene Kolben.

In einer besonders bevorzugten Ausführungsform besteht die Vorratseinrichtung (5) aus mehreren parallel zueinander verlaufenden, insbesondere aus zwei Transportbändern (10), welche jeweils zwischen mindestens zwei Rollen bzw. Rollenpaaren (11, 12) umlaufen, von denen mindestens eine Rolle bzw. Rollenpaar den Antrieb des betreffenden Transportbandes (10) bewirkt.

In einer weiteren besonders bevorzugten Ausführungsform enthalten die Klemmleisten (2) magnetisches, vorzugsweise ferromagnetisches Material, gegebenenfalls in Kombination mit nicht-magnetischem Material, und auf den Transportbändern (10) sind mehrere Magnete (13) angeordnet, beispielsweise Dauermagnete oder Elektromagnete, mit Hilfe derer die Klemmleisten (2) an den Transportbändern (10) fixiert werden können.

Die Klemmleisten (2) können aus der Vorratseinrichtung (5) durch eine Transportvorrichtung (6) entnommen werden. Dabei kann die Transportvorrichtung (6) sich sowohl in die jeweilige Position der Klemmleisten (2) in der Vorratseinrichtung (5) bewegen; vorzugsweise ist jedoch in der Vorratseinrichtung (5) eine Übernahmeposition (14) vorgesehen, an der die Klemmleisten (2) von der Transportvorrichtung (6) übernommen werden.

Anstelle der Klemmleisten (2) können auch einzelne Faserproben (4) aus den in der Vorratseinrichtung (5) befindlichen Klemmleisten (2) durch die Transporteinrichtung (6) entnommen werden.

In einer weiteren bevorzugten Ausführungsform weist die erfindungsgemäße Vorrichtung Sensorvorrichtungen (15) auf, mit deren Hilfe die Position der Klemmleisten (2) in der Vorratseinrichtung (5) bestimmbar ist.

Beispiele für Sensorvorrichtungen sind Lichtschranken, Kameras, Mikroschalter oder insbesondere Näherungsschalter.

Bei der Transportvorrichtung (6) für die Klemmleisten (2) oder die Faserproben (4) kann es sich um jede für diesen Zweck geeignete Vorrichtung handeln. Beispiele dafür sind Greifer, Spindelantriebe, Verfahrschlitten oder Roboterarme.

Besonders bevorzugt handelt es sich bei der Transportvorrichtung um eine hydraulisch oder vorzugsweise pneumatisch betriebene Mitnahmeeinrichtung (16), insbesonderen um einen Greifer (16a), der vorzugsweise pneumatisch betriebenen wird.

Für den Transport einzelner Faserproben (4) aus den in der Vorratseinrichtung (5) befindlichen Klemmleisten (2) in den Apparat (1) besitzt die Transportvorrichtung (6) vorzugsweise eine pneumatisch betriebene Einzelfaserklemme, welche mittels des Greifers (16a) bewegt wird. Der Transport zwischen Klemmleiste (2) und Apparat (1) kann beispielsweise mittels Linearführung oder Schwenkarm erfolgen.

In einer bevorzugten Ausführungsform der erfindungsgemäßen Vorrichtung weist der Apparat (1) eine Aufnahmeeinrichtung (17) auf, in welcher eine zur Messung vorgesehene Klemmleiste (2) mittels der Transportvorrichtung (6) abgelegt wird.

Diese Aufnahmeeinrichtung (17) weist vorzugsweise einen oder mehrere Anschläge (19) zum Positionieren der Klemmleiste (2) auf.

Die Aufnahmeeinrichtung (17) läßt sich ihrerseits wiederum im Apparat (1) positionieren, und ermöglicht somit eine Feineinstellung der Meßposition der Klemmleiste (2). Dieses Positionieren erfolgt vorzugsweise durch vertikale und horizontale Bewegung der Aufnahmeeinrichtung (17), wobei mittels der vertikalen Bewegung eine Grobeinstellung der Meßposition vorgenommen wird.

Besonders bevorzugt wird die Aufnahmeeinrichtung (17) mittels Spindeltrieb (18) in vertikaler Richtung bewegt.

In einer weiteren bevorzugten Ausführungsform der Erfindung sind der Apparat (1) und die Vorrichtung zum automatischen Bestücken des Apparates (1) mit einem Gehäuse (30) umgeben. Dadurch werden auf einfache Art und Weise Luftbewegungen vermieden, die für die Durchführung der Messung schädlich sein können.

Die Erfindung betrifft auch ein Verfahren zum automatischen Bestücken eines Apparates (1) zur Charakterisierung mechanischer und/oder geometrischer Eigenschaften von Stapelfaserproben umfassend folgende Maßnahmen:
a) Bestücken mehrerer Klemmleisten (2), die jeweils mehrere Klemmen (3) zur Aufnahme von Faserproben (4) aufweisen, mit Faserproben (4),
b) Einbringen der bestückten Klemmleisten (2) in Vorratseinrichtung (5), und
c) Verbringen der einzelnen Klemmleisten (2) oder einzelner Faserproben (4) mittels einer Transportvorrichtung (6) von der Vorratseinrichtung (5) automatisch in den Apparat (1).

Zur Probenvorbereitung werden die Klemmleisten (2) in üblicher Weise manuell mit Faserproben (4) bestückt.

Anschließend werden die mit Faserproben (4) bestückten Klemmleisten (2) manuell in die Vorratseinrichtung (5) eingebracht.

In einer bevorzugten Ausführungsform werden die Klemmleisten (2) in die aus mehreren parallel zueinander verlaufenden, insbesondere aus zwei Transportbändern (10) bestehende Vorratseinrichtung (5) durch Auflegen auf die Transportbänder (10) eingebracht.

In einer besonders bevorzugten Ausführungsform des erfindungsgemäßen Verfahrens werden die mit Klemmleisten (2) bestückten Transportbänder (10) durch Umlaufbewegung der Transportbänder und unter Kontrolle von Sensorvorrichtungen (15) in eine Anfangsposition bewegt, wobei die erste der zur Messung vorgesehenen Klemmleisten (2) in eine vorgegebene Übernahmeposition (14) bewegt wird.

In einer besonders bevorzugten Ausführungsform des erfindungsgemäßen Verfahrens werden die mit Klemmleisten (2) bestückten Transportbänder (10) durch Umlaufbewegung der Transportbänder bewegt und unter Kontrolle von Sensorvorrichtungen (15) werden die einzelnen zur Messung vorgesehenen Klemmleisten (2) in eine vorgegebene Übernahmeposition (14) bewegt, eine Mitnahmeeinrichtung (16) wird über der sich in der Übernahmeposition (14) befindlichen Klemmleiste (2) positioniert, und durch Absenken, Ergreifen, Anheben, Wegtransport in horizontaler Richtung und Ablage auf einer sich in einer Startposition (22) befindlichen Aufnahmeeinrichtung (17) wird die zur Messung vorgesehene Klemmleiste (2) aus der Übernahmeposition (14) in den Apparat (1) übertragen; oder durch Absenken, Ergreifen, Anheben, Wegtransport in horizontaler Richtung und Ablage in die Klemmen des Apparates (1) wird die zur Messung vorgesehene einzelne Faserprobe (4) aus der Übernahmeposition (14) in den Apparat (1) übertragen.

In einer weiteren bevorzugten Ausführungsform des erfindungsgemäßen Verfahrens wird die zur Messung vorgesehene und auf der Aufnahmeeinrichtung (17) abgelegte Klemmleiste (2) durch Absenken der Aufnahmeeinrichtung (17) von einer Startposition (22) auf eine der Höhe nach vorbestimmte Übergabeposition (21) verbracht und anschließend wird die Messung der einzelnen sich in der Klemmleiste (2) befindlichen Faserproben (4) im Apparat (1) vorgenommen, wobei vor den einzelnen Meßvorgängen die Faserproben (4) jeweils durch horizontale Bewegung der Aufnahmeeinrichtung (17) in eine definierte Meßposition (20) gebracht werden.

Die mit Faserproben (4) belegten Klemmen (3) der Klemmleiste (2) werden um eine vorbestimmte Strecke mittels einer Transportvorrichtung bewegt, so daß eine einzelne Faserprobe (4) in die Meßposition (20) des Apparates (1) zur Charakterisierung der mechanischen und/oder geometrischen Eigenschaften verbracht wird. Zu diesem Zweck wird die Klemme (3) in der Nähe der oberen Klemmbacke des Apparats (1) mittels einer Justiervorrichtung positioniert.

Von der Meßposition (20) aus kann die Faserprobe (4) in den Apparat (1) zur Charakterisierung der mechanischen und/oder geometrischen Eigenschaften übernommen werden. Zu diesem Zweck kann der Apparat (1) eine obere Klemme und gegebenenfalls eine untere Klemme zur Befestigung der zu charakterisierenden Faserprobe (4) aufweisen. Die obere Klemme ist dabei bewegbar und die Klemmleiste (2) ist mittels der Aufnahmeeinrichtung (17) so positioniert, daß die Klemmen (3) der Klemmleiste (2) sich in der Nähe der oberen Klemme des Apparats (1) vorbeibewegen lassen.

Anschließend wird die obere Klemme des Apparats (1) zur Charakterisierung der mechanischen und/oder geometrischen Eigenschaften mittels einer Transportvorrichtung bewegt, wobei die obere Klemme in geöffneter Position vorliegt; nach Erreichen der Faserprobe (4) wird die obere Klemme des Apparats (1) zur Charakterisierung der mechanischen und/oder geometrischen Eigenschaften mittels einer Öffnungs- und Schließvorrichtung unter Übernahme der Faserprobe (4) aus der Klemmleiste (2) geschlossen.

Danach wird die obere Klemme des Apparats (1) zur Charakterisierung der mechanischen und/oder geometrischen Eigenschaften zusammen mit der Faserprobe (4) mittels der Transportvorrichtung zur Charakterisierung der mechanischen und/oder geometrischen Eigenschaften der Faserproben (4) in den Apparat (1) bewegt.

Mit dem Apparat (1) zur Charakterisierung der mechanischen und/oder geometrischen Eigenschaften lassen sich Faserproben aller Art charakterisieren. Dabei kann es sich um ungekräuselte insbesondere jedoch um gekräuselte Faserproben handeln.

Beispiele für die Charakterisierung von mechanischen Eigenschaften sind die Aufnahme von Kraft-Dehnungs Diagrammen (KD-Diagrammen), die Messung des Schrumpfes, der Schrumpfkraft, der Kräuselung, der Einzelfaserfeinheit oder eine Kombination dieser Messungen.

Ein bevorzugtes Beispiel für die Charakterisierung der geometrischen Eigenschaften ist die Ermittlung der Zahl der Kräuselbögen von Stapelfasern.

Hinsichtlich des Einzelfasertiters und des faserbildenden Materials sind die erfindungsgemäße Vorrichtung bzw. erfindungsgemäße Verfahren keinen Beschränkungen unterworfen.

Typische Einzelfasertiter belaufen sich auf 1 bis 20 dtex.

Typische faserbildende Materialien sind Polyphenylensulfid, Polyetherketon, Glas oder carbonsiertes Polyacrylnitril (Kohlenstofffasern), Polyacrylnitril, Polyamide einschließlich der Aramide, und Polyester, insbesondere Polyethylenterephthalat.

Ein Beispiel für die Charakterisierung der mechanischen und/oder geometrischen Eigenschaften der Faserproben (4) mit dem Apparat (1) wird nachfolgend beschrieben. Dieses Beispiel betrifft die automatische Bestimmung der Anzahl der Kräuselbögen pro Längeneinheit von gekräuselten Stapelfasern.

Nach diesem Beispiel wird die Anzahl der Kräuselbögen pro Längeneinheit der Stapelfaserprobe mit dem Apparat (1) in folgender Weise bestimmt:
i) Erzeugung einer Abbildung vorgegebener Länge und vorgegebener Breite von der Stapelfaserprobe mittels einer in Richtung der Faserlängsachse bewegbaren Abbildungsvorrichtung,
ii) Erzeugung eines digitalen Rasters aus der Abbildung, dessen Pixel in Form von Zahlenwerten in einer Speichervorrichtung abgelegt werden, wobei die Zahlenwerte Maßzahlen für die Helligkeit an dem jeweiligen Ort der Abbildung darstellen, und
iii) Ermittlung der Anzahl der Kräuselbögen der abgebildeten Stapelfaserprobe aus dem digitalen Raster mittels digitaler Bildverarbeitung.

Das voranstehend skizzierte Verfahren kann mittels einer Abbildungsvorrichtung durchgeführt werden, die zur Erzeugung einer Abbildung vorgegebener Länge und vorgegebener Breite von der sich im Apparat (1) befindenden Stapelfaserprobe dient und die in Richtung der Faserlängsachse bewegbar ist und welche Abbildungsvorrichtung eine Datenverarbeitungsanlage ansteuert, welche ein digitales Raster aus der Abbildung erzeugt, dessen Pixel in Form von Zahlenwerten in der Speichervorrichtung abgelegt werden.

Zur Ermittlung der Anzahl der Kräuselbögen pro Längeneinheit kann wie folgt vorgegangen werden:
iv) Erzeugung einer Faserlängsachse in der Abbildung, welche dem Verlauf entspricht, den die Stapelfaserprobe in gestrecktem Zustand aufweisen würde,
v) Erzeugung einer Mittellinie in der Abbildung, welche Mittellinie in der Faserlängsachse nach Schritt iv) oder in vorgegebenem Abstand parallel zu besagter Faserlängsachse verläuft und die in Schritt i) erzeugte Abbildung der Stapelfaserprobe mindestens mehrfach schneidet, und
vi) Ermitteln der Anzahl der Schnittpunkte zwischen der in Schritt v) erzeugten Mittellinie und der in Schritt i) erzeugten Abbildung der Stapelfaserprobe als Maß für die Anzahl der in der Abbildung auftretenden Kräuselbögen der Stapelfaserprobe.

Bei der Abbildungsvorrichtung kann es sich beispielsweise um eine Zeilenkamera handeln.

Nach der Charakterisierung wird die obere Klemme des Apparats (1) mittels der Öffnungs- und Schließvorrichtung geöffnet und die Faserprobe (4) wird aus dem Apparat (1) zur Charakterisierung der mechanischen und/oder geometrischen Eigenschaften entfernt.

Nach der Charakterisierung der in der Klemmleiste (2) vorhandenen Faserproben (4) wird die Aufnahmeeinrichtung (17) zusammen mit der Klemmleiste (2) in einer bevorzugten Ausführungsform des erfindungsgemäßen Verfahrens in die Startposition (22) bewegt, die Klemmleiste (2) wird mittels der sich über der Aufnahmeeinrichtung (17) befindlichen Mitnahmeeinrichtung (16) durch Absenken dieser Mitnahmeeinrichtung (16), Ergreifen, Anheben, Wegtransport in horizontaler Richtung und Ablage der Klemmleiste (2) in die Übernahmeposition (14) der Transportbänder (10) in die Vorratseinrichtung (5) verbracht, und die mit Klemmleisten (2) bestückten Transportbänder (10) werden durch Umlaufbewegung der Transportbänder und unter Kontrolle von Sensorvorrichtungen (15) um eine vorbestimmte Strecke weiterbewegt, so daß die nächste zur Messung vorgesehene Klemmleiste (2) in die Übernahmeposition (14) einrückt.

Im Anschluß daran kann von der Transportvorrichtung (6) die nächste Klemmleiste (2) übernommen und nach dem oben skizierten Schema in den Apparat (1) zur Charakterisierung der mechanischen und/oder geometrischen Eigenschaften eingebracht werden.

Die folgenden Abbildungen erläutern die Erfindung.

In Figur 1 wird das Zusammenwirken von Vorratseinrichtung, Transportvorrichtung und Apparat zur Charakterisierung der mechanischen und/oder geometrischen Eigenschaften in perspektivischer Darstellung gezeigt.

In Figur 2 ist eine besonders bevorzugte Ausführungsform der Klemmleiste (2) dargestellt.

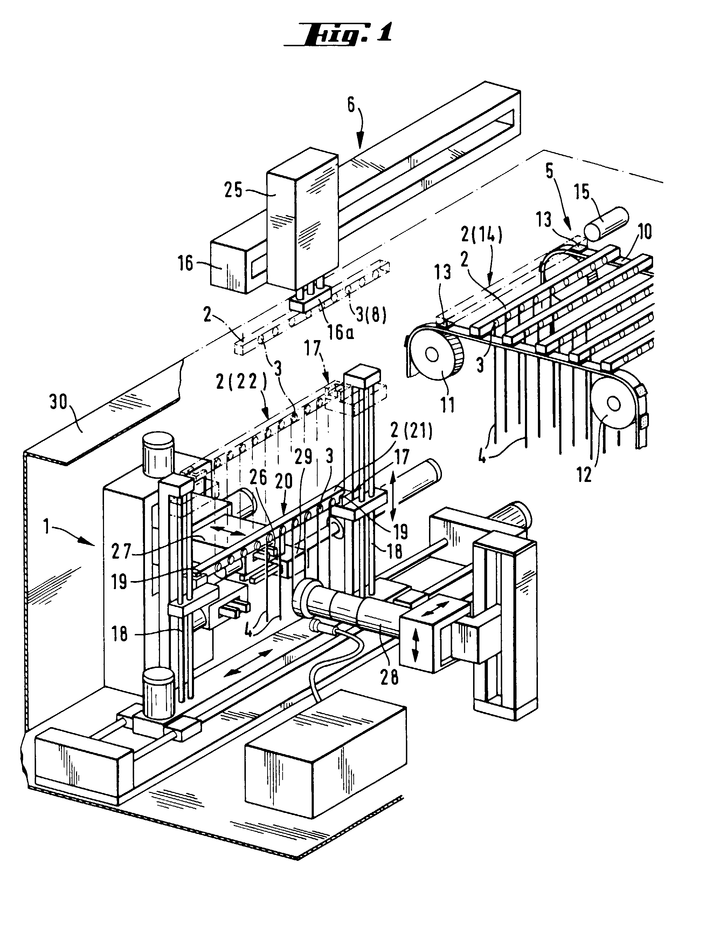

In Figur 1 ist eine Vorratseinrichtung (5) im Zusammenwirken mit einer Transporteinrichung (6) und dem Apparat (1) zur Charakterisierung von mechanischen und/oder geometrischen Eigenschaften dargestellt.

In der dargestellten Vorrichtung werden zwei mit Klemmleisten (2) bestückte und parallel zueinander verlaufende Transportbänder (10), welche die Vorratseinrichtung (5) bilden, durch Umlaufbewegung der Transportbänder und unter Kontrolle von Sensorvorrichtungen (15) in eine Übernahmeposition (14) bewegt. Die Klemmleisten (2) enthalten die zur Messung vorgesehenen Faserproben (4). Die Transportbänder (10) laufen zwischen zwei Rollen (11, 12) um, welche beide die Transportbänder antreiben. Die Klemmleisten (2) sind mit Hilfe von Magneten (13) auf den Transportbändern (10) fixiert. Von der Übernahmeposition (14) wird die betreffende Klemmleiste (2) mittels eines pneumatisch betriebenen Greifers (16a), welcher die Transportvorrichtung (6) darstellt, von der Vorratseinrichtung (5) in den Apparat (1) zur Charakterisierung mechanischer und/oder geometrischer Eigenschaften übergeführt. Greifer (16a) wird durch einen Schlitten (25) bewegt.

Der pneumatisch betriebene Greifer (16a) legt die Klemmleiste (2) in einer sich in einer Startposition (22) befindlichen Aufnahmeeinrichtung (17) ab. In der dargestellten Ausführungsform ist die Aufnahmeeinrichtung (17) mit einem Anschlag (19) zur Positionierung der Klemmleiste (2) versehen.

Die Aufnahmeeinrichtung (17) kann in vertikaler Richtung bewegt und so der Probenlänge angepaßt werden. Dazu wird die Aufnahmeeinrichtung (17) von der Startposition (22) auf eine der Höhe nach vorbestimmte Übergabeposition (21) abgesenkt. Dies erfolgt in der dargestellten Vorrichtung mittels eines Spindeltriebs (18). Durch horizonalen Vorschub der Aufnahmeeinrichtung (17) gelangen die Proben nacheinander in den Eingriffsbereich der Probenklemme (26). Die einzelnen Faserproben (4) werden zur Messung aus der Klemmleiste (2) in die Probenklemme (26) übernommen, welche mittels Schlitten (27) in horizontaler Richtung bewegt werden kann. Die Abbildung der Faserprobe erfolgt mittels der Kamera (28). Zusätzlich ist ein Entsorgungsgreifer (29) vorgesehen, mit dem die Faserprobe (4) nach der Messung aus dem Apparat (1) entfernt werden kann; anstelle des Entsorgungsgreifers kann das Entfernen der Faserprobe auch durch Absaugen erfolgen.

Nach der Messung der in der Klemmleiste (2) vorhandenen Faserproben (4) verläuft der oben skizzierte Verfahrensablauf in umgekehrter Reihenfolge; d.h. die Aufnahmeeinrichtung (17) wird zusammen mit der Klemmleiste (2) in die Startposition (22) bewegt, sodann wird die Klemmleiste (2) mittels des sich über der Aufnahmeeinrichtung (17) befindlichen pneumatisch betriebenen Greifers (16a) durch Absenken dieses Greifers, Ergreifen, Anheben, Wegtransport in horizontaler Richtung und Ablage der Klemmleiste (2) in die Übernahmeposition (14) der Transportbänder (10) in die Vorratseinrichtung (5) verbracht; sodann werden die mit Klemmleisten (2) bestückten Transportbänder (10) durch Umlaufbewegung der Transportbänder und unter Kontrolle von Sensorvorrichtungen (15) um eine vorbestimmte Strecke weiterbewegt, so daß die nächste zur Messung vorgesehene Klemmleiste (2) in die Übernahmeposition (14) einrückt.

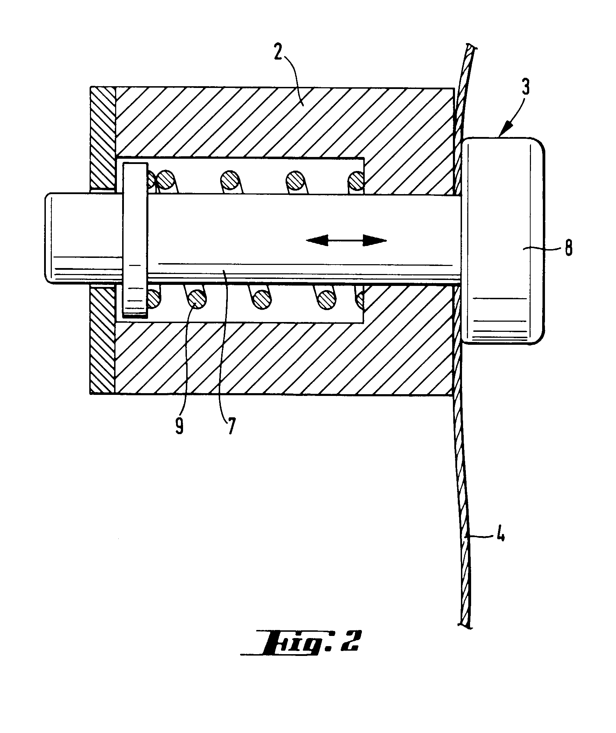

In Figur 2 ist eine besonders bevorzugte Ausführungsform einer Klemmleiste (2) dargestellt. Darin werden die Klemmen (3) durch Klemmbolzen (7) ausgebildet, die in Querrichtung durch die Klemmleiste (2) verlaufen und in dieser Querrichtung beweglich sind, sowie an einer Seite einen verbreiterten Kopf (8) aufweisen, welcher mittels einer im Innern der Klemmleiste angebrachten Feder (9) gegen die Klemmleiste (2) gedrückt wird. Diese Ausführungsform zeichnet sich durch einfache Handhabung, robuste Konstruktion sowie durch kostengünstige Fertigung aus.

## Patentansprüche

1. Vorrichtung zum automatischen Bestücken eines Apparates (1) zur Charakterisierung mechanischer und/oder geometrischer Eigenschaften von Stapelfaserproben umfassend folgende Elemente:
A) mehrere Klemmleisten (2), die jeweils mehrere Klemmen (3) zur Aufnahme von Faserproben (4) aufweisen,
B) mindestens eine Vorratseinrichtung (5) für die Klemmleisten (2), und
C) eine Transportvorrichtung (6), mit der die Klemmleisten (2) oder einzelne Faserproben (4) von der Vorratseinrichtung (5) automatisch in den Apparat (1) verbracht werden.

2. Vorrichtung nach Anspruch 1, dadurch gekennzeichnet, daß die Klemmen (3) durch Klemmbolzen (7) ausgebildet sind, die in Querrichtung durch die Klemmleiste (2) verlaufen und in dieser Querrichtung beweglich sind, an einer Seite einen verbreiterten Kopf (8) aufweisen, welcher mittels einer im Innern der Klemmleiste angebrachten Feder (9) gegen die Klemmleiste (2) gedrückt wird.

3. Vorrichtung nach Anspruch 1, dadurch gekennzeichnet, daß die Klemmleisten (2) in der Vorratseinrichtung (5) horizontal bewegbar sind.

4. Vorrichtung nach Anspruch 3, dadurch gekennzeichnet, daß die Vorratseinrichtung (5) aus mehreren parallel zueinander verlaufenden, insbesondere aus zwei Transportbändern (10) besteht, welche jeweils zwischen mindestens zwei Rollen (11, 12) umlaufen, von denen mindestens eine Rolle den Antrieb des betreffenden Transportbandes (10) bewirkt.

5. Vorrichtung nach Anspruch 4, dadurch gekennzeichnet, daß die Klemmleisten (2) aus magnetischem, vorzugsweise aus ferromagnetischem Material bestehen und auf den Transportbändern (10) mehrere Magnete (13) angeordnet sind, mit Hilfe derer die Klemmleisten (2) an den Transportbändern (10) fixiert werden können.

6. Vorrichtung nach Anspruch 3, dadurch gekennzeichnet, daß in der Vorratseinrichtung (5) eine Übernahmeposition (14) vorgesehen ist, an der die Klemmleisten (2) oder einzelne Faserproben (4) von der Transportvorrichtung (6) übernommen werden.

7. Vorrichtung nach Anspruch 1, dadurch gekennzeichnet, daß diese Vorrichtung Sensorvorrichtungen (15) aufweist, mit deren Hilfe die Position der Klemmleisten (2) in der Vorratseinrichtung (5) bestimmbar ist.

8. Vorrichtung nach Anspruch 7, dadurch gekennzeichnet, daß es sich bei den Sensorvorrichtungen (15) um Näherungsschalter handelt.

9. Vorrichtung nach Anspruch 1, dadurch gekennzeichnet, daß es sich bei der Transportvorrichtung (6) um eine hydraulisch oder vorzugsweise pneumatisch betriebene Mitnahmeeinrichtung (16), insbesonderen um einen Greifer (16a), handelt.

10. Vorrichtung nach Anspruch 9, dadurch gekennzeichnet, daß der Greifer (16a) eine pneumatisch betriebene Einzelfaserklemme aufweist.

11. Vorrichtung nach Anspruch 1, dadurch gekennzeichnet, daß der Apparat (1) eine Aufnahmeeinrichtung (17) aufweist, in welcher eine Klemmleiste (2) mittels der Transportvorrichtung (6) abgelegt wird.

12. Vorrichtung nach Anspruch 11, dadurch gekennzeichnet, daß die Aufnahmeeinrichtung (17) mittels Spindeltrieb (18) in vertikaler Richtung bewegbar ist.

13. Vorrichtung nach Anspruch 11, dadurch gekennzeichnet, daß die Aufnahmeeinrichtung (17) Anschläge (19) zum Positionieren der Klemmleiste (2) aufweist.

14. Vorrichtung nach Anspruch 11, dadurch gekennzeichnet, daß die Aufnahmeeinrichtung (17) in horizontaler Richtung bewegbar ist.

15. Vorrichtung nach Anspruch 1, dadurch gekennzeichnet, daß der Apparat (1) und die Vorrichtung zum automatischen Bestücken des Apparates (1) mit einem Gehäuse (30) umgeben sind.

16. Verfahren zum automatischen Bestücken eines Apparates (1) zur Charakterisierung mechanischer und/oder geometrischer Eigenschaften von Stapelfaserproben umfassend folgende Maßnahmen:
a) Bestücken mehrerer Klemmleisten (2), die jeweils mehrere Klemmen (3) zur Aufnahme von Faserproben (4) aufweisen, mit Faserproben (4),
b) Einbringen der bestückten Klemmleisten (2) in Vorratseinrichtung (5), und
c) Verbringen der einzelnen Klemmleisten (2) oder einzelner Faserproben (4) mittels einer Transportvorrichtung (6) von der Vorratseinrichtung (5) automatisch in den Apparat (1).

17. Verfahren nach Anspruch 16, dadurch gekennzeichnet, daß die mit Faserproben (4) bestückten Klemmleisten (2) manuell in eine Vorratseinrichtung (5) eingebracht werden, die aus mehreren parallel zueinander verlaufenden, insbesondere aus zwei Transportbändern (10) besteht, welche jeweils zwischen mindestens zwei Rollen (11, 12) umlaufen, von denen mindestens eine Rolle den Antrieb des betreffenden Transportbandes (10) bewirkt, wobei die Klemmleisten (2) auf die Transportbänder (10) gelegt werden.

18. Verfahren nach Anspruch 17, dadurch gekennzeichnet, daß die mit mit Klemmleisten (2) bestückten Transportbänder (10) durch Umlaufbewegung der Transportbänder und unter Kontrolle von Sensorvorrichtungen (15) in eine Anfangsposition bewegt werden, wobei die erste der zur Messung vorgesehenen Klemmleisten (2) in eine vorgegebene Übernahmeposition (14) bewegt wird.

19. Verfahren nach Anspruch 17, dadurch gekennzeichnet, daß die mit Klemmleisten (2) bestückten Transportbänder (10) durch Umlaufbewegung der Transportbänder und unter Kontrolle von Sensorvorrichtungen (15) die einzelnen zur Messung vorgesehenen Klemmleisten (2) in eine vorgegebene Übernahmeposition (14) bewegt werden, daß eine Mitnahmeeinrichtung (16a) über der sich in der Übernahmeposition (14) befindlichen Klemmleiste (2) positioniert wird, durch Absenken, Ergreifen, Anheben, Wegtransport in horizontaler Richtung und Ablage auf einer sich in einer Startposition (22) befindlichen Aufnahmeeinrichtung (17) die zur Messung vorgesehene Klemmleiste (2) aus der Übernahmeposition (14) in den Apparat (1) übertragen wird.

20. Verfahren nach Anspruch 19, dadurch gekennzeichnet, daß die zur Messung vorgesehene und auf der Aufnahmeeinrichtung (17) abgelegte Klemmleiste (2) durch Absenken der Aufnahmeeinrichtung (17) auf eine der Höhe nach vorbestimmte Meßposition (20) verbracht wird und anschließend die Messung der einzelnen sich in der Klemmleiste (2) befindlichen Faserproben (4) im Apparat (1) erfolgt, wobei vor den einzelnen Meßvorgängen die Faserproben (4) jeweils durch horizontale Bewegung der Aufnahmeeinrichtung (17) in eine definierte Meßposition (20) gebracht werden.

21. Verfahren nach Anspruch 20, dadurch gekennzeichnet, daß nach der Messung der in der Klemmleiste (2) vorhandenen Faserproben (4) die Aufnahmeeinrichtung (17) in die Startposition (22) bewegt wird, die Klemmleiste (2) mittels der sich über der Aufnahmeeinrichtung (17) befindlichen Mitnahmeeinrichtung (16a) durch Absenken dieser Mitnahmeeinrichtung (16a), Ergreifen, Anheben, Wegtransport in horizontaler Richtung und Ablage der Klemmleiste (2) in die Übernahmeposition (14) der Transportbänder (10) in die Vorratseinrichtung (5) verbracht wird, daß die mit Klemmleisten (2) bestückten Transportbänder (10) durch Umlaufbewegung der Transportbänder und unter Kontrolle von Sensorvorrichtungen (15) um eine vorbestimmte Strecke weiterbewegt werden, so daß die nächste zur Messung vorgesehene Klemmleiste (2) in die Übernahmeposition (14) einrückt.

22. Verfahren nach Anspruch 17, dadurch gekennzeichnet, daß die mit Klemmleisten (2) bestückten Transportbänder (10) durch Umlaufbewegung der Transportbänder und unter Kontrolle von Sensorvorrichtungen (15) die einzelnen zur Messung vorgesehenen Klemmleisten (2) in eine vorgegebene Übernahmeposition (14) bewegt werden, daß eine Mitnahmeeinrichtung (16a) über der sich in der Übernahmeposition (14) befindlichen Klemmleiste (2) positioniert wird, durch Absenken, Ergreifen, Anheben, Wegtransport in horizontaler Richtung und Ablage in die Klemmen des Apparates (1) die zur Messung vorgesehene einzelne Faserprobe (4) aus der Übernahmeposition (14) in den Apparat (1) übertragen wird.

## Claims

1. A device for automatically feeding an apparatus (1) for characterising mechanical and/or geometrical properties of samples of staple fibres and comprising the following elements:
A) a plurality of clamping strips (2) each of which comprises a plurality of clamps (3) for receiving fibre specimens (4),
B) at least one supply means (5) for the clamping strips (2), and
C) a transporting device (6) by which the clamping strips (2) or individual fibre specimens (4) are automatically conveyed from the supply means (5) and into the apparatus (1).

2. A device according to claim 1, characterised in that the clamps (3) are formed by clamping bolts (7) which extend in a transverse direction through the clamping strip (2) and which are adapted for movement in this transverse direction and have on one side a widened head (8) which is pressed against the clamping strip (2) by means of a spring (9) mounted inside the clamping strip.

3. A device according to claim 1, characterised in that the clamping strips (2) are adapted for horizontal movement in the supply means (5).

4. A device according to claim 3, characterised in that the supply means (5) consist of a plurality of, extending parallel with one another and in particular of two conveyor belts (10), which respectively revolve between at least two rollers (11, 12) of which at least one roller provides the drive for the respective conveyor belt (10).

5. A device according to claim 4, characterised in that the clamping strips (2) consist of magnetic, preferably ferromagnetic, material and in that there are on the conveyor belts (10) a plurality of magnets (13) by means of which the clamping strips (2) can be fixed on the conveyor belts (10).

6. A device according to claim 3, characterised in that in the supply means (5) there is a take-over position (14) at which the clamping strips (2) or individual fibre samples (4) are taken over by the transporting device (6).

7. A device according to claim 1, characterised in that this device comprises sensor devices (15) by means of which the position of the clamping strips (2) in the supply means (5) can be determined.

8. A device according to claim 7, characterised in that the sensor devices (15) are proximity switches.

9. A device according to claim 1, characterised in that where the transporting device (6) is concerned, this is an entraining means (16), particularly a gripper (16a) which can be hydraulically or preferably pneumatically operated.

10. A device according to claim 9, characterised in that the gripper (16a) comprises a pneumatically operated single fibre clamp.

11. A device according to claim 1, characterised in that the apparatus (1) comprises a receiving means (17) in which a clamping strip (2) is deposited by means of the transporting device (6).

12. A device according to claim 11, characterised in that the receiving means (17) is adapted to be moved in a vertical direction by a spindle drive (18).

13. A device according to claim 11, characterised in that the receiving means (17) has abutments (19) for positioning of the clamping strip (2).

14. A device according to claim 11, characterised in that the receiving means (17) is adapted for movement in a horizontal direction.

15. A device according to claim 1, characterised in that the apparatus (1) and the device for automatically feeding the apparatus (1) are enclosed by a housing (30).

16. A method for automatically feeding an apparatus (1) for characterising mechanical and/or geometrical properties of stable fibre samples and comprising the following measures:
a) feeding of a plurality of clamping strips (2) each of which comprises a plurality of clamps (3) for accommodating fibre samples (4) with fibre samples (4)
b) introduction of the loaded clamping strips (2) into the supply means (5), and
c) bringing of the individual clamping strips (2) or of individual fibre samples (4) automatically into the apparatus (1) by means of a transporting device (6) from the supply means (5).

17. A method according to claim 16, characterised in that the clamping strips (2), loaded with fibre samples (4), are brought manually into a supply means (5) which consists of a plurality of and in particular two conveyor belts (10) which extend parallel with each other and which respectively revolve between at least two rollers (11, 12) of which at least one roller produces the drive for the respective conveyor belt (10), the clamping strips (2) being placed on the conveyor belts (10).

18. A method according to claim 17, characterised in that the conveyor belts (10) which are loaded with clamping strips (2) are moved by the revolving movement of the conveyor belts and under the control of the sensor devices (15) into an initial position whereby the first clamping strips (2) which are to be measured are moved into a predetermined take-over position (14).

19. A method according to claim 17, characterised in that the conveyor belts (10) which are loaded with clamping strips (2) are, by the revolving motion of the conveyor belts and under the control of the centre devices (15), brought into a predetermined take-over position (14) and in that an entraining means (16a) is positioned over the clamping strip (2) which is in the take-over position (14) and, by lowering, gripping, lifting, transporting away in a horizontal direction and deposition on a receiving means (17) which is in a starting position (22), the clamping strip (2) which is intended to be measured is conveyed out of the take-over position (14) and into the apparatus (1).

20. A method according to claim 19, characterised in that the clamping strip (2) which is intended to be measured and which is deposited on the receiving means (17) is, by a lowering of the receiving means (17), brought to a measuring position (20) which is determined according to the height after which measurement of the individual fibre samples (4) which are disposed in the clamping strip (2) takes place in the apparatus (1) whereby, prior to the individual measuring processes, the fibre specimens (4) are respectively and by horizontal movement of the receiving means (17) brought into a defined measuring position (20).

21. A method according to claim 20, characterised in that after measurement of the fibre samples (4) present in the clamping strip (2) the receiving means (17) is moved into the starting position (22), the clamping strip (2) is by means of the entraining means (16a) disposed above the receiving means (17) is, by lowering of this entraining means (16a) gripping, lifting, transporting away in a horizontal direction and deposition of the clamping strip (2) into the take-over position (14) of the conveyor belts (10), brought into the supply means (5) and in that, loaded with clamping strips (2), the conveyor belts (10) are, by the revolving movement of the conveyor belts and under the control of sensor devices (15), moved on by a predetermined distance so that the next clamping strip (2) which is intended to be measured moves into the take-over position (14).

22. A method according to claim 17, characterised in that the conveyor belts (10) loaded with clamping strips (2) are, by a rotating movement of the conveyor belts, and, under control of sensor devices (15), the individual clamping strips (2) which are intended to be measured, brought into a predetermined take-over position (14) and in that an entraining means (16a) is positioned above the clamping strip (2) which is in the take-over position (14) and, by lowering, gripping, raising, carrying away in a horizontal direction and deposition into the clamps of the apparatus (1), the individual fibre sample (4) which is intended to be measured is carried out of the take-over position (14) and into the apparatus (1).

## Revendications

1. Dispositif de chargement automatique d'un appareil (1) destiné à caractériser les propriétés mécaniques et/ou géométriques des échantillons de fibres discontinues, comprenant les éléments suivants :
A) plusieurs baguettes de blocage (2) qui sont munies chacune de plusieurs organes de serrage (3) destinés à recevoir les échantillons de fibres (4),
B) au moins un dispositif de stockage (5) pour les baguettes de blocage (2), et
C) un dispositif de transport (6), avec lequel les baguettes de blocage (2) ou des échantillons de fibres (4) isolés sont amenés automatiquement du dispositif de stockage (5) vers l'appareil (1).

2. Dispositif selon la revendication 1, caractérisé en ce que les organes de serrage (3) sont formés par des boulons de serrage (7), qui traversent la baguette de blocage (2) dans le sens transversal et sont mobiles dans ce sens transversal, comportent sur un côté une tête (8) élargie, qui est poussée contre la baguette de blocage (2) par un ressort (9) monté à l'intérieur de la baguette de blocage.

3. Dispositif selon la revendication 1, caractérisé en ce que les baguettes de blocage (2) peuvent se déplacer horizontalement dans le dispositif de stockage (5).

4. Dispositif selon la revendication 3, caractérisé en ce que le dispositif de stockage (5) est formé par plusieurs bandes de transport (10), en particulier deux, parallèles les unes aux autres, qui tournent chacune entre au moins deux poulies (11, 12), parmi lesquelles au moins une poulie assure l'entraînement de la bande de transport (10) concernée.

5. Dispositif selon la revendication 4, caractérisé en ce que les baguettes de blocage (2) sont réalisées dans un matériau magnétique, de préférence un matériau ferromagnétique, et plusieurs aimants (13) sont disposés sur les bandes de transport (10), au moyen desquels les baguettes de blocage (2) peuvent être bloquées contre les bandes de transport (10).

6. Dispositif selon la revendication 3, caractérisé en ce qu'il est prévu une position de transfert (14) dans le dispositif de stockage (5), dans laquelle les baguettes de blocage (2) ou des échantillons de fibres (4) isolés sont réceptionnés par le dispositif de transport (6).

7. Dispositif selon la revendication 1, caractérisé en ce que ledit dispositif comporte des dispositifs de capteurs (15), au moyen desquels il est possible de déterminer la position des baguettes de blocage (2) dans le dispositif de stockage (5).

8. Dispositif selon la revendication 7, caractérisé en ce que les dispositifs de capteurs (15) utilisés sont des capteurs de présence.

9. Dispositif selon la revendication 1, caractérisé en ce que le dispositif de transport (6) est un dispositif d'entraînement (16) actionné par voie hydraulique ou, de préférence, par voie pneumatique, en particulier une griffe (16a).

10. Dispositif selon la revendication 9, caractérisé en ce que la griffe (16a) est munie d'une pince pour fibre isolée, actionnée par voie pneumatique.

11. Dispositif selon la revendication 1, caractérisé en ce que l'appareil (1) comporte un dispositif de réception (17), dans lequel est déposée une baguette de blocage (2), au moyen du dispositif de transport (6).

12. Dispositif selon la revendication 11, caractérisé en ce que le dispositif de réception (17) se déplace dans le sens vertical par l'intermédiaire d'un mécanisme de transmission par broches (18).

13. Dispositif selon la revendication 11, caractérisé en ce que le dispositif de réception (17) est muni de butées (19) destinées à définir la position de la baguette de blocage (2).

14. Dispositif selon la revendication 11, caractérisé en ce que le dispositif de réception (17) peut se déplacer dans le sens horizontal.

15. Dispositif selon la revendication 1, caractérisé en ce que l'appareil (1) et le dispositif de chargement automatique de l'appareil (1) sont entourés par un carter (30).

16. Procédé de chargement automatique d'un appareil (1) destiné à caractériser les propriétés mécaniques et/ou géométriques des échantillons de fibres discontinues, comprenant les opérations suivantes :
a) chargement des échantillons de fibres (4) sur plusieurs baguettes de blocage (2), qui comportent chacune plusieurs organes de serrage (3) destinés à recevoir les échantillons de fibres (4),
b) introduction des baguettes de blocage (2) chargées dans le dispositif de stockage (5), et
c) transfert automatique de chaque baguette de blocage (2) ou chaque échantillon de fibre (4) du dispositif de stockage (5) vers l'appareil (1), au moyen d'un dispositif de transport (6).

17. Procédé selon la revendication 16, caractérisé en ce que les baguettes de blocage (2), portant les échantillons de fibres (4), sont introduites manuellement dans un dispositif de stockage (5), qui est formé par plusieurs bandes de transport (10), en particulier deux, parallèles les unes aux autres, lesquelles tournent entre au moins deux poulies (11, 12) parmi lesquelles au moins une poulie assure l'entraînement de la bande de transport (10) concernée, les baguettes de blocage (2) étant posées sur les bandes de transport (10).

18. Procédé selon la revendication 17, caractérisé en ce que les bandes de transport (10), portant les baguettes de blocage (2), sont amenées dans une position initiale par le mouvement de rotation des bandes de transport et sous le contrôle de dispositifs de capteurs (15), la première des baguettes de blocage (2) prévues pour le processus de mesure étant déplacée dans une position de transfert (14) prédéfinie.

19. Procédé selon la revendication 17, caractérisé en ce que les bandes de transport (10), portant les baguettes de blocage (2), sont déplacées par le mouvement de rotation des bandes de transport et chacune des baguettes de blocage (2) prévues pour la mesure est déplacée sous le contrôle de dispositifs de capteurs (15) dans une position de transfert (14) prédéfinie, en ce qu'un dispositif d'entraînement (16a) est positionné ensuite au-dessus de la baguette de blocage (2) placée dans la position de transfert (14), et la baguette de blocage (2) prévue pour la mesure est transférée par l'intermédiaire d'un déplacement vers le bas, d'un mouvement de saisie, d'un déplacement vers le haut, d'un transport en sens horizontal et de la dépose sur un dispositif de réception (17) qui se trouve en position de départ (22), à partir de sa position de transfert (14) vers l'appareil (1).

20. Procédé selon la revendication 19, caractérisé en ce que la baguette de blocage (2), prévue pour la mesure et déposée sur le dispositif de réception (17) est amenée, par un mouvement vers le bas du dispositif de réception (17) vers une position de mesure (20), prédéfinie en fonction de la hauteur, et ensuite les échantillons de fibres (4) individuels qui se trouvent dans la baguette de blocage (2) sont mesurés dans l'appareil (1), les échantillons de fibres (4) étant amenés chacun avant les différents processus de mesure dans une position de mesure (20) définie, au moyen d'un déplacement horizontal du dispositif de réception (17).

21. Procédé selon la revendication 20, caractérisé en ce que, à la fin du processus de mesure des échantillons de fibres (4) placés dans la baguette de blocage (2), le dispositif de réception (17) se déplace vers la position de départ (22), la baguette de blocage (2) est amenée par le dispositif d'entraînement (16a), placé au-dessus du dispositif de réception (17), vers le dispositif de stockage (5), par l'intermédiaire du déplacement vers le bas de ce dispositif d'entraînement (16a), un mouvement de saisie, un déplacement vers le haut, un transport en sens horizontal et la dépose de la baguette de blocage (2) dans la position de transfert (14) des bandes de transport (10), en ce que les bandes de transport (10), portant les baguettes de blocage (2), sont déplacées ensuite sur une distance prédéfinie, par le mouvement de rotation des bandes de transport et sous le contrôle de dispositifs de capteurs (15), de telle sorte que la prochaine baguette de blocage (2) prévue pour le processus de mesure est avancée dans la position de transfert (14).

22. Procédé selon la revendication 17, caractérisé en ce que les bandes de transport (10) portant les baguettes de blocage (2), sont déplacées par le mouvement de rotation des bandes de transport et chacune des baguettes de blocage (2) prévues pour le processus de mesure est déplacée sous le contrôle de dispositifs de capteurs (15) vers une position de transfert (14) prédéfinie, en ce que le dispositif d'entraînement (16a) est positionné au-dessus de la baguette de blocage (2) placée dans la position de transfert (14), et l'échantillon de fibre (4) isolé, prévu pour le processus de mesure, est transféré par l'intermédiaire d'un déplacement vers le bas, d'un mouvement de saisie, d'un déplacement vers le haut, d'un transport en sens horizontal et de la mise en place dans les pinces de l'appareil (1) à partir de sa position de transfert (14) vers l'appareil (1).
